# EUROPEAN PATENT APPLICATION

(11) **EP 1 946 756 A1**
(43) Date of publication of application: **23.07.2008**
(21) Application number: 07100668.8
(22) Date of filing: 17.01.2007
(51) Int. Cl.: A61K 31/277, A61K 8/41, A61P 37/00, A61P 17/00, A61Q 19/00

(54) **Use of entacapone in cosmetic, dermatological and pharmaceutical compositions**

(71) Applicant: Revotar Biopharmaceuticals AG, 16761 Henningsdorf (DE)
(72) Inventor: Kranich, Remo, 13503, Berlin (DE); Aydt, Ewald M., 14163, Berlin (DE); Bock, Daniel, 30161, Hannover (DE); Wolff, Gerhard, 16548, Glienicke-Nordbahn (DE)
(74) Representative: Jacobi, Markus Alexander

(57) **Abstract**

A compound of formula (I), a salt of this compound or an isomeric or polymorphic form thereof can be used for the preparation of a cosmetic or dermatological composition, which is useful for the treatment or prophylaxis of itching or skin ageing caused by a combination of intrinsic and extrinsic factors.

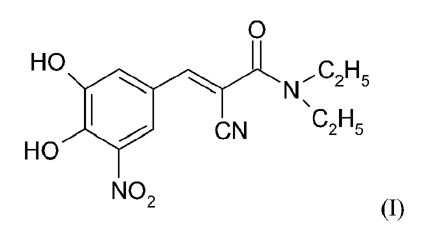

## Description

The present invention relates to the use of Entacapone for the treatment, prophylaxis, and/or diagnosis of micro-inflammatory, inflammatory, T-cell mediated disorders, and viral skin diseases and tumors of the skin.

Cell-adhesion molecules (CAMs) like E-, P-, and L-selectin are part of a complex cascade leading to the migration of circulating white blood cells (leukocytes) from the blood stream into the surrounding tissue (extravasation). Physiologically, leukocyte extravasation is of critical importance for homeostasis and immuno-surveillance of living beings including humans. Lymphocytes for example, are constitutively leaving the blood stream into lymphatic tissues in order to patrol for harmful antigens. Under pathological circumstances however, e.g. local or systemic inflammation and/or injury of the vascular system, this fundamental process is dys-regulated, at least in part, due to an increased surface expression of the adhesion molecules E- and P-selectin. Consequently, the excessive leukocyte extravasation leads to a pathological cellular infiltrate with subsequent tissue damage in several clinically relevant settings.

Disease states such as acute lung injury (ALI), acute respiratory distress syndrome (ARDS), asthma bronchiale (asthma), chronic obstructive pulmonary disease (COPD), psoriasis, rheumatoid arthritis, and sepsis are all associated with tissue inflammation induced and perpetuated by pathologically activated leukocytes infiltrating the respective tissue. In addition, exaggerated leukocyte infiltration contributes to the pathogenesis of ischemic-reperfusion injury (IRI) associated with organ transplantation, cardiopulmonary bypass or percutaneous transluminal angioplasty.

To extravasate, leukocytes interact with three different families of CAMs in a sequential and concerted action:

At first with selectins ( see G. S. Kanas et al., Blood, 1996, 88, 3259-3287), at second with integrins (see E.A. Clark et al., Science, 1995, 268, 233-239) and at third with members of the immunoglobulin superfamily (see A.F. Williams et al., Annu. Rev. Immunol., 1988, 6, 381-405]. Based on the general multi-step paradigm [K. Ley, Results Probl. Cell Differ., 2001, 33, 177-200). The ordered sequence of steps leading to transmigration can be summarized in general as follows: Free-flowing leukocytes pass post-capillary venules with high velocities.

In order to leave the vasculature at points of inflammation they have to be captured and slowed down (mediated by selectins) allowing them to finally stop and to firmly adhere to the endothelial wall (mediated by integrins) and finally to transmigrate from circulation into the surrounding tissue. Where the diameter of capillaries is smaller than the diameter of leukocytes (as seen in the lung) , L-selectin mediated retention is involved in addition to mechanical hindrance [see D. Bock et al., Curr. Respir. Medicine Rev. 2006, 2, 339-354].

Selectins are also involved in signal transduction, which results in the activation of endothelial cells and/or leukocytes, for instance [A. Zarbock et al., J. Clin. Invest. 2006, 116(12), 3211-3219]. Signal transduction via selectins can be induced through binding of large as well as small molecules to selectins [B. Brenner et al., PNAS 1996, 93, 15376-15381].

The selectin family of adhesion molecules is comprised of three structurally related calcium-dependent carbohydrate binding cell surface proteins, E-, P-, and L-selectin. E-selectin is expressed on inflamed endothelium, P-selectin is expressed on inflamed endothelium as well as on platelets and L-selectin is expressed on leukocytes. Selectins are composed of an amino terminal lectin domain, an epidermal growth factor (EGF)-like domain, a variable number of complement receptor-related repeats, a hydrophobic transmembrane domain and a C-terminal cytoplasmic domain. The binding interactions leading to the adhesion of the leukocytes are supposed to be mediated by contacts of the lectin domain of the selectins and various carbohydrate ligands on the surface of the leukocytes.

All three selectins can bind with low affinity to the carbohydrate sialyl Lewis X (sLe^{X}), a glycosyl moiety present on the surface of most leukocytes. A structurally related glycosyl moiety, sialyl Lewis a (sLe^{a}), is predominantly found on the surface of cancer cells [see K. Okazaki et al., J. Surg. Res., 1998, 78(1), 78-84].

In case of P-selectin, a distinct high affinity glycoprotein ligand has been described [R.P. McEver, R.D. Cummings, J.Clin.Invest., 1997, 100, 485-492], the so-called P-selectin glycoprotein ligand-1 (PSGL-1), which contributes to a high affinity selectin binding by its sLe^{X} moiety as well as by parts of its peptide components, in particular sulphated tyrosine residues. PSGL-1 is one of the most important selectin ligands binding with highest affinity to P-selectin, but it also binds to E- and L-selectin. It is a homo-dimeric sialomucin predominantly expressed on leukocytes.

In inflammatory diseases, dys-regulated extravasation is, at least in part, mediated due to an increased cell surface expression of E- and P-selectin. In contrast to their low basal expression, E- and P-selectin expression is upregulated during inflammation, leading to a substantial recruitment of leukocytes into the inflamed tissue. Although selectin-mediated cell adhesion is required for fighting infection, there are various situations in which such cell adhesion is undesirable or excessive, resulting in severe tissue damage instead of repair. In the case of many acute as well as chronic inflammatory disorders (e.g., asthma, COPD, psoriasis, etc.), an association between infiltration of activated leukocytes into the tissue simultaneously with a marked elevation of tissue expression of corresponding adhesion molecules, particularly E- and P-selectin, has been demonstrated (see Muller et al., J. Pathol., 2002, 198(2), 270-275).

Leukocyte infiltration may also play a role in inflammatory symptoms in the course of transplant and graft rejection. Also the process of blood clotting is further promoted by leukocyte-leukocyte and leukocyte-platelet binding, which occurs because leukocytes possess both L-selectin and its corresponding ligand P-glycoprotein ligand-1 (PSGL-1) and can thus interact with themselves via PSGL-1, and they can also bind to platelets which carry P-selectin. In addition, selectins are involved in micro-inflammatory processes causing ageing of the skin [P. U. Giacomoni et al., Micron 2004, 35, 179-184].

Selectins play also a crucial role in micro-inflammatory processes, which are responsible for ageing of the skin. The signs of ageing of the skin resulting from the effects on the skin of intrinsic and extrinsic factors are defined by the appearance of wrinkles and fine lines, by the yellowing of the skin which develops a wizened appearance along with the appearance of pigmentation blemishes, by a change in the thickness of the skin, generally resulting in a thickening of the stratum corneum and of the epidermis and a thinning of the dermis, by disorganization of the elastin and collagen fibers which causes a loss of elasticity, of suppleness and of firmness, and by the appearance of telnagiectasia.

Some of these signs are more particularly associated with intrinsic or physiological ageing, that is so to say with "normal" ageing associated with age, whereas others are more specific to extrinsic ageing, that is so to say ageing caused by the environment in general; such ageing is more particularly photo-ageing due to exposure to the sun, to light or to any other radiation. Other factors causing ageing of the skin are atmospheric pollution, wounds, infections, traumatisms, anoxia, cigarette smoke, hormonal status, neuropeptides, electromagnetic fields, gravity, lifestyle (e.g. excessive consumption of alcohol), repetitive facial expressions, sleeping positions, and psychological stressors.

The changes in the skin, which occur due to intrinsic ageing, are the consequence of a genetically programmed sequence involving endogenous factors. This intrinsic ageing in particular causes slowing down of the regeneration of skin cells, which is reflected essentially in the appearance of clinical damage such as a reduction of the subcutaneous adipose tissue and the appearance of fine lines or small wrinkles, and in histopathological changes such as an increase in the number and thickness of the elastic fibers, a loss of vertical fibers from the elastic tissue membrane and the presence of large irregular fibroblasts in the cells of this elastic tissue.

In contrast, extrinsic ageing results in clinical damage such as thick wrinkles and the formation of flabby and weather-beaten skin, and in histopathological changes such as an excessive accumulation of elastic substance in the upper dermis and degeneration of the collagen fibers.

Intrinsic and extrinsic factors of ageing of the skin share common mechanisms [P. U. Giacomoni et al., Biogerontology 2004, 2, 219-229]. These factors trigger a process leading to the accumulation of damages in the skin resulting in skin ageing since the expression of cell adhesion molecules provokes recruitment and diapedesis of circulating immune cells, which digest the extracellular matrix (ECM) by secreting collagenases, myeloperoxidases and reactive oxygen species. The activation of these lytic processes provokes random damage of these resident cells, which in turn secrete prostaglandins and leukotrienes.

These signaling molecules induce the degranulation of resident mast cells which release the autacoid histamine and the cytokine TNFalpha thus activating endothelial cells lining adjacent capillaries which release P-selectin and the synthesis of cell adhesion molecules such as E-selectin and ICAM-1. This closes a self-maintained micro-inflammatory cycle, which results in the accumulation of ECM damage, i.e. skin ageing.

In the pathology of T-cell mediated diseases activated T-cells play a pivotal role. They orchestrate deleterious cellular and humoral reactions through the secretion of immunoregulatory mediators and via cell-cell contact. Modulating T-cell activation or influencing the reactivity of the T-cell after activation is considered a promising strategy to treat T-cell mediated disorders, such as asthma or psoriasis. For instance, Efalizumab a recombinant humanized antibody binding to CD11a a subunit of leukocyte-function antigen 1 (LFA-1) expressed on T-cells blocks activation of T-cells, trafficking of T-cells to sites of inflammation and T-cell reactivity and is effective in psoriatic patients [see J. C. Cather et al., Expert Opin. Biol. Ther., 2005, 5, 393-403].

Alfacept is a fusion protein containing a domain of leukocyte-function antigen 3 (LFA-3). By binding to CD2 on T-cells Alefacept is inhibiting T-cell activation by blocking the contact between T-cells and antigen presenting. In addition the Fc portion of Alefacept by binding to Fc-receptors on accessory cells such as natural killer cells or macrophages is causing apoptosis and depletion of T-cells. Alefacept is effective in psoriatic patients.

Papilloma viruses are a DNA virus infecting mammalian human epithelial cells which cause uncontrolled cell replication. There are many types of papilloma virus infecting human and animal species, but they all can infect the basal epithelial cells and persist in episomal or as DNA integrated into the host genome. The effect of papilloma virus include genital warts (Condylomata acuminate), common and plantar warts, bovine papillomas, and cervical intra-epithelial neplasia in woman. The human papilloma virus (HPV) of type HPV6 and HPV11 are the ones most commonly detected and because HPV 16 and HPV 18 have been detected in malignant squamous cell carcinoma from cancer of the penis, cancer of the cervix and Condyloma acuminate, there is a strong possibility that HPV 16 and HPV 18 are linked to the malignancy of Condyloma acuminate.

There is a strong medical, cosmetic (including skin care), and dermatological need for novel highly potent anti-inflammatory and anti-micro-inflammatory compounds for the treatment, prophylaxis, and/or diagnosis of various indications or conditions where inflammatory or micro-inflammatory conditions play a role.

Most of the available anti-inflammatory pharmaceutical therapies, which are available on the market, comprise corticosteroids or NSAIDs (non steroidal anti-inflammatory drugs) having several serious drawbacks/side effects, and target different steps of the inflammatory cascade. There have been various investigations to develop low-molecular weight compounds with a modulatory effect on selectin mediated processes. These compounds include disalicylates and disalicylate-based C-glycosides [WO 99/29706], benzyl amino sulfonic acids [WO 03/097658], diglycosylated 1,2-diols [WO 97/01569], substituted 5-membered heterocycles [WO 00/33836], mannopyranosyloxy-phenyl-benzoic acids [EP-A 0758243], piperazine based compounds [US 6432957B1], gallic acid derivatives of peptides [WO 2004/018502], gallic acid [C. C. M. Appeldoorn et al., Circulation 2005, 111, 106-112; EP-A 1481669], and quinic acid derivatives [N. Kaila et al., J. Med. Chem. 2005, 48, 4346-4357 ].

However, none of these selectin-antagonizing compounds have successfully passed clinical trials up to date [S. J. Romano, Treat. Respir Med 2005, 4(2), 85-94; M. P. Schön, Therapeutics and Clinical Risk Management, 2005, 1(3), 201-208]. Most of the compounds developed so far have high molecular weights and often bear carbohydrates and/or peptides making them prone to degradation and modification by peptidases and/or glycosidases. Carbohydrate-bearing structures have further disadvantages such as high degree of chirality, anomericity, and low probability of transport through lipid bilayers. Similar disadvantages are known for peptide-bearing compounds. Some other compounds developed for antagonizing selectin mediated processes contain pyrogallol-substructures.

The leading compound in the field of selectin antagonists is bimosiamose [S. J. Romano, Treat. Respir Med 2005, 4(2), 85-94]. Presently, bimosiamose [D. Bock et al., New Drugs, 2003, D04, 28, p.28; EP 0 840 606 B1] is the most advanced compound in clinical studies. It is, however, a high molecular weight compound with carbohydrate structures.

Various cosmetic and dermatological compositions (including for skin care) intended inter alia to prevent or treat ageing of the skin are known. Retinoic acid and derivatives thereof have been described as anti-ageing agents in skin care, cosmetic, or dermatological compositions, in particular in US 4603146. Hydroxy acids such as lactic acid, glycolic or alternatively citric acid are also known for this same application, these acids having been described in numerous patents and publications (e.g. EP-A-413528) and introduced into numerous skin care, cosmetic, or dermatological compositions on the market.

Aromatic ortho-hydroxy acids such as salicylic acid have also been proposed (e.g. WO 93/10756 and WO 93/10755). All of these compounds act against ageing of the skin by desquamation, that is to say removal of the dead cells at the surface of the stratum corneum. This desquamation is also referred to as a keratolytic property. However, these compounds also have side effects, consisting of stinging and redness, which the user finds unpleasant. Thus, there remains a need for anti-ageing agents which are at least as effective as the known compounds, but do not exhibit their drawbacks.

The medical need for selective immuno-intervention in T-cell mediated disorders is very high, as the available immunosuppressive drugs are substantially inadequate because of limited efficacy, modest selectivity, and considerable toxicity. Available immunosupressive drugs like glucocorticoids, cytostatics or drugs acting on immunophilins (e.g. Cylosporin A) that are used to dampen T-cell reactivity in T-cell mediated diseases are frequently associated with severe side effects like hypertension, dyslipidemia, hyperglycemia, peptic ulcers, liver and kidney injury. Because of non-selective immunosupression the majority of them have the potential to increase the risk of infection and spreading of malignant cells.

Unlike the established strategies to treat, prevent, and/or diagnose inflammatory or micro-inflammatory indications or conditions, modulating the function of selectins is a novel concepts intervening the micro-/inflammation cascade at a very early stage and treating, preventing, and/or diagnosing inflammatory or micro-inflammatory indications or conditions according to the present inventions represents a strategy without the drawbacks known from other strategies.

There is also a strong medical need for the treatment of Condyloma acuminata caused by human papilloma viruses. Presently, physical methods are predominately used for the treatment of genital warts caused by human papilloma viruses.

These methods include surgical removal, electrocauterization, cryosurgery, and laser therapy, for instance. An additional medicinal treatment is the use of Podophyllin, Bleomycin, Imquimod, Interferon, 5-Fluorouracil, for instance. However, surgical treatment is very unpleasant for the patient and can lead to further infections. Topical treatments have the risk of side effects.

These medications work either by cytotoxic tissue destruction or by enhancing the cellular immune response by causing local inflammation. Therefore no satisfactory treatment is presently available.

Surprisingly, it turned out that Entacapone ((*2E*)-2-cyano-3-(3,4-dihydroxy-5-nitrophenyl)-*N,N*-diethylacrylamide, compound of formula (I)), an orally bioavailable and well-tolerated nitrocatechol, inhibits *in vitro* attachment of leukocytes to E- and P-selectin.

In addition, it turned out that the compound of formula (I) shows improved *in vitro* activity for P- and L-selectin in a PSGL-1-like setting as compared to sLe^{X}. The compound of formula (I) can be considered as selectin antagonist which modulates processes where selectins are involved. Furthermore, it surprisingly turned out that the compound of formula (I) modulates T-cells *in vitro.* With compound of formula (I) pre-incubated T-cells showed reduced attachment to E- and P-selectin, clearly indicating the modulation of T-cells through compound of formula (I). The compound of formula (I) can also be used to treat skin diseases caused by human papilloma viruses, such as Condyloma acuminate.

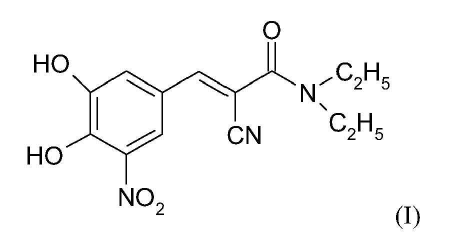

Compound of formula (I), a marketed small molecule drug with a relative molecular weight below 500, has been developed for the treatment of Morbus Parkinson acting as specific catechol-*O*-methyl transferase (COMT) inhibitors (see P. T. Männisto et al., Pharmacological Reviews 1999, 51, 593-628; EP-A 00426468). Nitro-catechols, such as compound of formula (I), Nitecapone, and Tolcapone have also been described to have cardioprotective function caused by iron-chelating [N. Haramaki et al., Biochemical Pharmacology 1995, 50, 839-843] or decreasing plasma homocysteine levels [E. Nissinen et al., J. Neural Transm. 2005, 112, 1213-1221]. Nitecapone has also been reported to act as radical scavenger [see Y. J. Suzuki et al., Free Radical Biology & Medicine, 1992, 13, 517-525].

A recent published individual observation indicated that the compound of formula (I) may protect from angiotensin II-induced renal tissue damage and the authors speculated that this effect is mediated through antioxidative and anti-inflammatory mechanism. As the authors showed, the beneficial effect of the compound of formula (I) was present only in rats which were genetically modified to over express a cDNA of human renin and human angiotensin genes (dTGR-model). In addition, it was shown that the observed beneficial effect was only observed in the kidney but not in the heart, the second target organ for angiotensin II induced tissue damage [T. Helkamaa et al., J. Hypertens., 2003, 21, 2353-2363]. It clearly demonstrated that the suggested beneficial effect of the compound of formula (I) which may protect from angiotensin II-induced tissue damage is restricted to the kidney and could not be demonstrated in the second target organ angiotensin II-induced tissue damage, the heart.

However, nitrochatechols, in particular the compound of formula (I), have neither been described as selectin modulators nor as compounds modulating the extravasation of leukocytes nor as T-cell modulating compounds so far.

Unlike most of the sLe^{X}-mimicking compounds developed in this field, the compound of formula (I) is not prone to glycosidases or peptidases. Most of the selectin antagonists developed so far are structurally and biologically based on the properties of sLe^{X} or sLe^{a}. The resulting compounds showed, therefore, low biological activity like sLe^{X} or sLe^{a}. This invention, however, provides a new potent small and drug-like selectin antagonists that has been invented on the basis of biological *in vitro* assays mimicking high affinity PSGL-1 and PSGL-1-like ligands or any ligands bearing sLe^{X} or sLe^{a} and tyrosinesulfate motifs [N. V. Bovin; Biochem Soc Symp.; 2002;(69):143-60. N. V. Bovin; Glycoconj. J.; 1998; 15(5); 431-46. T.V. Pochechueva et al.; Bioorg Med Chem Lett.; 2003;13(10);1709-12. G. Weitz-Schmidt et al.; Anal. Biochem.;1996; 238; 184-190].

The compound of formula (I) presents the ability of modulating cell adhesion and modulate selectin- as well as PSGL-1-like mediated binding. The compound has the ability to modulate the interaction of selectins with sLe^{X}/sLe^{a} and also the interaction between selectins and tyrosinesulfate residues. As compared to sLe^{X}, the compound of formula (I) shows increased biological activity. Furthermore, the compound of formula (I) modulates the state and function of T-cells surprisingly. By directly targeting T-cells the compound of formula (I) influences their ability to adhere to adhesion molecules under flow shear conditions. T-cells (Jurkat) pre-incubated with compound of formula (I) were less effective in adhering to immobilized selectins although free compound was removed prior to the adhesion assay (see Example 3).

The ability of T-cells to bind to endothelial cells or other leukocytes via adhesion molecules plays a key role in the pathology of T-cell mediated disorders. In addition, compound of formula (I) is known to be an orally bioavailable drug, which is well-tolerated. The compound of formula (I) is useful for the treatment, prophylaxis, and/or diagnosis of acute and chronic micro-inflammatory, inflammatory, and T-cell-mediated disorders or conditions, as well as other medical conditions where selectin mediated, cell adhesion molecule mediated as well as T-cell mediated processes play a role.

Surprisingly the compound of formula (I) shows high penetration in a skin-penetration assay (see Example 4). Taking its skin penetration characteristics as well as its good systemic tolerance in humans into account, the compound of formula (I) is suitable for cosmetic and dermatological applications.

Thus, the present invention relates to the use of a compound of formula (I), a salt of compound of formula (I) or an isomeric or polymorphic form thereof for the preparation of a cosmetic or dermatological composition. The preferred isomeric form is the structure shown above, but other stereo isomeric forms and structural isomeric forms of the compound of formula (I) are also of interest.

The amount used of compound of formula (I), a salt of compound of formula (I) or an isomeric or polymorphic form thereof corresponds to the amount required to obtain the desired result using the cosmetic or dermatological compositions.

One skilled in this art is capable of evaluating this effective amount, which depends on the derivative used, the individual on whom it is applied, and the time of this application. To provide an order of magnitude, in the cosmetic or dermatological compositions according to the invention, the compound of formula (I), a salt of compound of formula (I) or an isomeric or polymorphic form thereof may be administered in an amount representing from 0.001% to 40% by weight, preferentially 0.005% to 30% by weight and more preferentially from 0.01% to 20% by weight.

A further aspect covers cosmetic compositions comprising a compound of formula (I), a salt of compound of formula (I) or an isomeric or polymorphic form thereof and at least one cosmetically tolerable component, e.g. a cosmetically tolerable component for skin applications

The amounts of the various components of the physiological medium of the cosmetic composition according to the invention are those generally included in the fields under consideration. When the cosmetic composition is an emulsion, the proportion of the fatty phase may range from 2% to 80% by weight and preferably from 5% to 50% by weight relative to the total weight of the cosmetic composition.

The aqueous phase is adjusted as a function of the content, in the fatty phase, of compound of formula (I), a salt of compound of formula (I) or an isomeric or polymorphic form thereof, of alcohol and nonionic surfactant and also that of the optional additional ingredients, to obtain 100% by weight. In practice, the aqueous phase represents from 5% to 90% by weight. The fatty phase may contain fatty or oily compounds, which are liquid at room temperature (25°C.), generally known as oils, waxes and pasty or semi-solid products. These oils may be mutually compatible and may form a macroscopically homogeneous liquid fatty phase. The aqueous phase contains water and optionally a water-miscible ingredient, for instance polyols such as propylene glycol, glycerol or sorbitol.

In particular, the cosmetic composition often contains one or more components such as nonionic surfactants. The nonionic surfactant or the mixture of nonionic surfactants with a hydrophilic-lipophilic balance (HLB) of greater than 10 is (are) preferably used in an amount sufficient to dissolve with the compound of formula (I) or an isomeric or polymorphic form thereof.

In practice, this nonionic surfactant or mixture of nonionic surfactants may be included in the compositions of the invention in a concentration ranging from 0.01% to 10% by weight, preferentially from 0.05% to 5% by weight and more preferentially from 0.1% to 2% by weight.

More specifically, cosmetic compositions of the present invention contain nonionic surfactants having a HLB of greater than 10 and which may be up to 20. These are compounds that are well known per se [see Handbook of Surfactants by M. R. Porter, published by Blackie & Son (Glasgow and London), 1991, pp. 116-178]. Thus, they may be selected especially from among polyethoxylated, polypropoxylated or polyglycerolated fatty acids, (C1-C20) alkylphenols, alpha-diols and alcohols, which are preferably hydrogenated, with a fatty chain containing, for example, from 8 to 22 carbon atoms, the mean number of ethylene oxide or propylene oxide structural units optionally ranging especially from 3.5 to 200 (for example from 5 to 100) and the number of glycerol groups optionally ranging especially from 2 to 100 (for example from 3 to 50), and mixtures thereof. Also exemplary are copolymers of ethylene oxide and propylene oxide, condensates of ethylene oxide and propylene oxide on fatty alcohols; polyethoxylated fatty amides and preferably those containing on average from 3.5 to 200 mol of propylene oxide and/or ethylene oxide; polyglycerolated fatty amides and preferably those containing on average from 1.5 to 40; ethoxylated fatty acid esters of sorbitan especially containing from 2 to 30 mol on average of ethylene oxide and a fatty chain especially containing from 8 to 22 (for example from 12 to 18) carbon atoms; fatty acid esters of sucrose; fatty acid esters of polyethylene glycol; (C6-C24)alkylpolyglycosides; N-(C6-C24) alkylglucamine derivatives; amine oxides such as(C10-C14) alkylamine oxides; and mixtures thereof.

In the cosmetic compositions an alcohol may be used alone or as a mixture and is selected from among C1-C4 alcohols such as ethanol or isopropanol, and mixtures thereof. The C1-C4 alcohol is preferably included in an amount sufficient to dissolve with the nonionic surfactant the compound of formula (I) or an isomeric or polymorphic form thereof. In practice, the C1-C4 alcohol or the mixture of C1-C4 alcohols may be included in the cosmetic compositions of the invention in a concentration ranging from 2% to 80% of the total weight of the composition, preferentially from 10% to 70% and more preferentially from 20% to 60% by weight relative to the total weight of the cosmetic composition.

The cosmetic compositions of the invention are preferentially for cosmetic applications and in particular for topical application to the skin.

Thus, the cosmetic composition should contain a non-toxic physiologically acceptable medium that can be applied to human skin. For a topical application to the skin, the cosmetic composition may be in the form of a solution, a suspension, an emulsion or a dispersion of more or less fluid consistency and especially liquid or semi-liquid consistency, obtained by dispersing a fatty phase in an aqueous phase (O/W) or, conversely, (W/O), or alternatively a gel. A cosmetic composition in the form of a mousse or in the form of a spray or an aerosol then comprising a pressurized propellant may also be provided. Also the compositions may be in the form of a haircare lotion, a shampoo or hair conditioner, a liquid or solid soap, a treating mask, or a foaming cream or gel for cleansing the hair. They may also be in the form of a hair dye or hair mascara.

The cosmetic compositions of the invention may also comprise one or more other ingredients usually employed in the fields under consideration, selected from among formulation additives, for instance aqueous-phase or oily-phase thickeners or gelling agents, dyestuffs that are soluble in the medium of the cosmetic composition, solid particles such as mineral or organic fillers or pigments in the form of microparticles or nanoparticles, preservatives, fragrances, hydrotopes or electrolytes, neutralizers (acidifying or basifying agents), propellants, anionic, cationic or amphoteric surfactants, polymers, in particular water-soluble or water-dispersible anionic, nonionic, cationic or amphoteric film-forming polymers, mineral or organic salts, chelating agents; mixtures thereof.

These additives may be present in the cosmetic composition in the amounts generally employed in cosmetics, and especially in a proportion of from 0.01% to 50% and preferably from 0.1 % to 20%, for example from 0.1% to 10%, of the total weight of the cosmetic composition. Oils that may be included according to the invention may be made of oils of mineral origin (liquid petroleum jelly or hydrogenated isoparaffin), oils of plant origin (liquid fraction of shea butter, sunflower oil, apricot oil, soybean oil, fatty alcohol or fatty acid), oils of animal origin (perhydrosqualene), synthetic oils (fatty acid esters or purcellin oil), silicone oils (linear or cyclic polydimethylsiloxanes, and phenyl trimethicones) and fluoro oils (perfluoropolyethers).

Waxes that may be mentioned include silicone waxes, beeswax, candelilla wax, rice bran wax, carnauba wax, paraffin wax or polyethylene wax. When the cosmetic composition is in emulsion form, it also contains one or more emulsifiers and optionally one or more co-emulsifiers generally employed in cosmetics. Their nature also depends on the sense of the emulsion. In practice, the emulsifier and, optionally, the co-emulsifier are present in the cosmetic composition in a proportion ranging from 0.1% to 30% by weight, preferably from 0.5% to 20% by weight and better still from 1% to 8% by weight. The emulsion may also contain lipid vesicles and especially liposomes. As thickeners or gelling agents that may be included according to the invention, mention may be made of thickening or gelling polymers, for instance crosslinked polyacrylic acids, associative polymers and non-polymeric thickeners or gelling agents, for instance modified or unmodified clays, amides and metal salts of fatty acids.

Beside cosmetically inactive ingredients the cosmetic compositions of the present invention may also comprise one or more cosmetically active ingredients with beneficial action on the skin. Thus, the present invention relates to cosmetic compositions comprising a compound of formula (I), a salt of compound of formula (I) or an isomeric or polymorphic form thereof and at least one further cosmetically active ingredient.

As cosmetically/dermatologically/pharmaceutically active agents with beneficial action the following are cited: of active agents selected from among: UV-blocking agents, such as sunscreens; vitamins (A, C or E) and derivatives thereof (retinyl palmitate, tocopheryl acetate or tocopheryl palmitate); ceramides; proteins and protein hydrolysates, peptides and amino acids; urea and allantoin; sugars and sugar derivatives, for instance reduced or oxidized sugars; extracts of plant origin (those from Iridacea plants or from soybean) or of bacterial origin; hydroxy acids, in particular hydroxycarboxylic acids or ketocarboxylic acids (fruit acid, salicylic acid) and esters thereof, for instance 5-n-octanoylsalicylic acid; diazoxide, spiroxazone, or phospholipids, for instance lecithin; additional active compounds , especially: nicotinic acid esters, among which especially are tocopheryl nicotinate, benzyl nicotinate and C1-C22 alkyl nicotinates such as methyl or hexyl nicotinate; pyrimidine derivatives, for instance 2,4-diamino-6-piperidinopyrimidine 3-oxide or "Minoxidil" pyrimidine 3-oxide derivatives, for instance those described in WO 92/01437 or WO 96/09048, and especially "Aminexil" or 2,4-diaminopyrimidine 3-N-oxide; anti-androgens, for instance steroidal or non-steroidal inhibitors of 5-[alpha]-reductase, such as finasteride and the compounds described in US 5516779, cyprosterone acetate, azeleic acid, its salts and its derivatives and the compounds described in US 5480913, flutamide and the compounds described in US 5411981, US 5565467 and US 4910226, prostaglandins and analogues thereof, for instance latanoprost; anti-bacterial, anti-fungal or anti-dandruff agents, for instance selenium derivatives, ketoconazole, octopirox, triclocarban, triclosan, zinc pyrithione, itraconazole, asiatic acid, hinokitiol, mipirocine, tetracyclines, especially erythromycin and the compounds described in EP-A 0,680,745, clinycine hydrochloride, benzoyl peroxide or benzyl peroxide and minocycline; calcium-channel antagonists and potassium-channel agonists; hormones; steroidal anti-inflammatory agents, for instance glucocorticoids, corticosteroids (for example: hydrocortisone) and non-steroidal anti-inflammatory agents, for instance glycyrrhetinic acid and [alpha]-bisabolol, benzydamine and the compounds described in EP-A 0770399, WO-94/06434;

Retinoid RXR receptor agonists and retinoid antagonists; free-radical scavengers and antioxidants, for instance butylhydroxyanisole or butylhydroxytoluene; anti-seborrhoeic agents; anti-parasitic agents; anti-viral agents; anti-pruriginous agents; carotenoids, for instance [beta]-carotene; lactones and the corresponding salts thereof; essential fatty acids, for instance linoleic acid, eicosatetraenoic acid, linolenic acid and eicosatrienoic acid, or esters and amides thereof; essential oils; phenols and polyphenols, for instance flavonoids; and mixtures thereof. This additional active agent may be for example in an amount of from 0.001 % to 10% by weight, preferably from 0.1% to 5% and better still from 0.5% to 3% by weight.

Dermatological compositions comprising a compound of formula (I), a salt of compound of formula (I) or an isomeric or polymorphic form thereof and at least one dermatologically tolerable component, e.g. a dermatologically tolerable component for skin applications, are also subject of the invention.

Dermatologically tolerable components that can be used for the dermatological compositions described here are identical to the cosmetically tolerable components as defined in this invention.

In addition to dermatologically inactive ingredients the dermatological compositions may also comprise dermatologically or pharmaceutically active ingredients.

Thus, the present invention also relates to dermatological compositions comprising a compound of formula (I), a salt of compound of formula (I) or an isomeric or polymorphic form thereof and at least one further dermatologically or pharmaceutically active ingredient. The dermatologically or pharmaceutically active ingredients that can be used for the dermatological compositions described herein are defined as the cosmetically active ingredients defined above. Dermatologically or pharmaceutically active ingredients can be identical to the cosmetically active ingredients as defined in this invention.

Another embodiment of this invention is a process for the preparation of a cosmetic composition by mixing a compound of formula (I), a salt of compound of formula (I) or an isomeric or polymorphic form thereof, at least one cosmetically tolerable component and eventually further cosmetically active ingredients.

In particular, a process for the preparation of a cosmetic composition by mixing a compound of formula (I), a salt of compound of formula (I) or an isomeric or polymorphic form thereof, at least one cosmetically tolerable component and eventually further cosmetically active ingredients, wherein the composition includes from 0.01% to 20% by weight of compound of formula (I), a salt of compound of formula (I) or an isomeric or polymorphic form thereof, based on the total weight of the composition is subject of this invention.

A further aspect of this invention deals with a process for the preparation of a dermatological composition by mixing a compound of formula (I), a salt of compound of formula (I) or an isomeric or polymorphic form thereof, at least one dermatologically tolerable component and eventually further pharmaceutically active ingredients.

In particular, a process for the preparation of a dermatological composition by mixing a compound of formula (I), a salt of compound of formula (I) or an isomeric or polymorphic form thereof, at least one dermatologically tolerable component and eventually further pharmaceutically active ingredients, wherein the composition includes from 0.01% to 20% by weight of compound of formula (I), a salt of compound of formula (I) or an isomeric or polymorphic form thereof, based on the total weight of the composition is subject of the present invention.

The cosmetic and dermatological compositions contain, for example, compound of formula (I), a salt of compound of formula (I) or an isomeric or polymorphic form thereof and a cosmetically, or dermatologically tolerable component. The cosmetic and dermatological preparations can be manufactured using standard technologies, known to the person skilled in the art. For this, the compound of formula (I), a salt of compound of formula (I) or an isomeric or polymorphic form thereof according to the invention are brought into a suitable dosage form together with one or more cosmetic or dermatological vehicles and/or inactive ingredients and, if the preparation of a combination preparation is desired, one or more other pharmaceutical, cosmetic, or dermatological active compounds having therapeutic, or prophylactic action, which can then be used as cosmetic or dermatological product.

The compound of formula (I) shows in vitro activity in inflammation and micro-inflammation-related in vitro assays (see Examples 1 and 2). Thus, another embodiment of the invention is the use of the compound of formula (I), a salt of compound of formula (I) or an isomeric or polymorphic form thereof for the preparation of a cosmetic composition for the treatment or prophylaxis of micro-inflammatory conditions.

The use of a compound of formula (I), a salt of compound of formula (I) or an isomeric or polymorphic form thereof in combination with a further cosmetically active compound for the preparation of a cosmetic composition for the treatment or prophylaxis of micro-inflammatory conditions, is also subject of the invention. Cosmetically active compounds beneficial for cosmetic applications have already been defined above.

Micro-inflammatory processes cause skin ageing where selectins play a crucial role. Thus, inhibition of selectin mediated function is a promising strategy to treat and/or prevent skin ageing. The present invention, relates to the use of a compound of formula (I), a salt of compound of formula (I) or an isomeric or polymorphic form thereof for the preparation of a cosmetic composition for the treatment or prophylaxis of itching or skin-ageing caused by intrinsic factors.The signs of ageing of the skin resulting from the effects on the skin of intrinsic and extrinsic factors are defined by the appearance of wrinkles and fine lines, by the yellowing of the skin which develops a wizened appearance along with the appearance of pigmentation blemishes, by a change in the thickness of the skin, generally resulting in a thickening of the stratum corneum and of the epidermis and a thinning of the dermis, by disorganization of the elastin and collagen fibers which causes a loss of elasticity, of suppleness and of firmness, and by the appearance of telnagiectasia.

The changes in the skin, which occur due to intrinsic ageing, are the consequence of a genetically programmed sequence involving endogenous factors. This intrinsic ageing in particular causes slowing down of the regeneration of skin cells, which is reflected essentially in the appearance of clinical damage such as a reduction of the subcutaneous adipose tissue and the appearance of fine lines or small wrinkles, and in histopathological changes such as an increase in the number and thickness of the elastic fibers, a loss of vertical fibers from the elastic tissue membrane and the presence of large irregular fibroblasts in the cells of this elastic tissue.

A further embodiment of the invention is the use of a compound of formula (I), a salt of compound of formula (I) or an isomeric or polymorphic form thereof for the preparation of a dermatological composition for the treatment or prophylaxis of micro-inflammatory diseases or conditions.

In particular the invention relates to the use of a compound of formula (I), a salt of compound of formula (I) or an isomeric or polymorphic form thereof in combination with a further dermatologically or pharmaceutically active compound for the preparation of a dermatological composition for the treatment or prophylaxis of micro-inflammatory diseases or conditions. The dermatologically or pharmaceutically active ingredients that can be used for the dermatological compositions described herein are defined as the cosmetically active ingredients defined above. Dermatologically or pharmaceutically active ingredients can be identical to the cosmetically active ingredients as defined in this invention.

A preferred embodiment of the invention is the use of a compound of formula (I), a salt of compound of formula (I) or an isomeric or polymorphic form thereof for the preparation of a dermatological composition for the treatment or prophylaxis of itching or skin-ageing caused by extrinsic factors. Extrinsic ageing of the skin results in clinical damage such as thick wrinkles and the formation of flabby and weather-beaten skin, and in histopathological changes such as an excessive accumulation of elastic substance in the upper dermis and degeneration of the collagen fibers.

Extrinsic factors include environmental factors in general; more particularly photo-ageing due to exposure to the sun, to light or to any other radiation, atmospheric pollution, wounds, infections, traumatisms, anoxia, cigarette smoke, hormonal status as response to external factors, neuropeptides, electromagnetic fields, gravity, lifestyle (e.g. excessive consumption of alcohol), repetitive facial expressions, sleeping positions, and psychological stressors.

Ageing of the skin may also be caused by a combination of intrinsic and extrinsic factors. Therefore, the present invention also relates to the use of a compound of formula (I), a salt of compound of formula (I) or an isomeric or polymorphic form thereof for the preparation of a dermatological composition for the treatment or prophylaxis of itching or skin ageing caused by a combination of intrinsic and extrinsic factors.

The use of a compound of formula (I), a salt of compound of formula (I) or an isomeric or polymorphic form thereof for the preparation of a dermatological composition for the treatment or prophylaxis of viral skin diseases and /or tumor diseases or conditions, is also subject of this invention.

A further aspect of the invention is the use of a compound of formula (I), a salt of compound of formula (I) or an isomeric or polymorphic form thereof in combination with a further dermatologically active compound for the preparation of a dermatological composition for the treatment or prophylaxis of viral skin diseases and /or tumor diseases or conditions. The dermatologically active ingredients that can be used for the dermatological compositions described herein are defined as the cosmetically active ingredients defined above. Dermatologically or pharmaceutically active ingredients can be identical to the cosmetically active ingredients as defined in this invention.

A further aspect of the present invention is the use of a compound of formula (I) or an isomeric or polymorphic form thereof for the preparation of a dermatological composition for the treatment or prophylaxis of viral skin diseases caused by papilloma viruses, in particular human papilloma viruses (HPV), by herpes viruses, varicella zoster virus, or human herpes virus.

A further aspect of the invention is the use of a compound of formula (I), a salt of compound of formula (I) or an isomeric or polymorphic form thereof for the preparation of a dermatological composition for the treatment or prophylaxis of viral skin diseases caused by papilloma viruses, in particular by HPV 1, 2, 3, 4, 5, 8, 9, 11, 12, 13, 14, 15, 16, 17, 18, 19-29, 31, 32, 34, 36-38, 46-50, 56, 58, by herpes viruses, in particular herpes simplex virus 1, herpes simplex virus 2, or human herpes virus 1, 2, 3, 4, 7, or 8.

A further aspect of the invention is the use of a compound of formula (I), a salt of compound of formula (I) or an isomeric or polymorphic form thereof for the preparation of a dermatological composition for the treatment or prophylaxis of viral skin diseases, in particular genital warts, benign tumors of the skin and/or mucosa caused by papilloma viruses, in particular verrucae plantares, verrucae vulgares, verrucae planae juveniles, epidermodysplasia verruciformis, Condylomata acuminate, Condylomata plana, bowenoid papulosis, papilloma on the larynx and oral mucosa, focal epithelial hyperplasia, herpes labialis, Kaposi's sarcoma, varicella and shingles.

The compound of formula (I) surprisingly modulates the state of T-cells(see Example 3). Thus, another embodiment of the invention is the use of a compound of formula (I), a salt of compound of formula (I) or an isomeric or polymorphic form thereof for the preparation of a pharmaceutical composition for the treatment, diagnosis, or prophylaxis of T-cell-mediated diseases or conditions.

The use of a compound of formula (I), a salt of compound of formula (I) or an isomeric or polymorphic form thereof in combination with a further pharmaceutically active compound for the preparation of a pharmaceutical composition for the treatment, diagnosis, or prophylaxis of T-cell-mediated diseases or conditions, is also subject of the present invention. The pharmaceutically active ingredients that can be used for the pharmaceutical compositions described herein are defined as the cosmetically active ingredients defined above. Dermatologically or pharmaceutically active ingredients can be identical to the cosmetically active ingredients as defined in this invention.

In particular, the present invention relates to the use of a compound of formula (I), a salt of compound of formula (I) or an isomeric or polymorphic form thereof for the preparation of a pharmaceutical composition for the treatment, diagnosis, or prophylaxis of T-cell mediated disorders.

These disorder are in particular the following diseases: asthma bronchiale, psoriasis, atopische dermatitis, systemic lupus erythematosus (SLE), Sjörgen's syndrome, rheumatoid arthritis, encephalitis and in particular acute disseminated encephalomyelitis (ADEM), Addison's disease, antiphospholipid antibody syndrome (APS), aplastic anemia, autoimmune hepatitis, coeliac disease, inflammatory bowel disease and in particular Crohn's disease, diabetes mellitus (type 1), Goodpasture's syndrome, hyperthyroidism and in particular Graves' disease, Guillain-Barré syndrome (GBS; also called acute inflammatory demyelinating polyneuropathy, acute idiopathic polyradiculoneuritis, acute idiopathic polyneuritis and Landry's ascending paralysis), hypothyroidism and in particular Hashimoto's disease, idiopathic thrombocytopenic purpura, lupus erythematosus, multiple sclerosis, myasthenia gravis, opsoclonus myoclonus syndrome (OMS), optic neuritis, thyroiditis and in particular Ord's thyroiditis, pemphigus, polyarthritis, primary biliary cirrhosis, psoriasis, rheumatoid arthritis, Reiter's syndrome, Sjögren's syndrome, Takayasu's arteritis, temporal arteritis, warm autoimmune hemolytic anemia, vasulitis and in particular Wegener's granulomatosis, alopecia universalis, Behcet's disease, Chagas' disease, chronic fatigue syndrome, dysautonomia including postural orthostatic tachycardia syndrome (POTS), endometriosis, hidradenitis suppurativa, interstitial cystitis, neuromyotonia, sarcoidosis, scleroderma, ulcerative colitis, vitiligo, vulvodynia.

The Examples 1 and 2 illustrate the use of the compound of formula (I) for the treatment, diagnosis, and prophylaxis of inflammatory disorders. The present invention relates to the use of a compound of formula (I), a salt of compound of formula (I) or an isomeric or polymorphic form thereof for the preparation of a pharmaceutical composition for the treatment, diagnosis, or prophylaxis of inflammatory diseases or conditions.

The use of a compound of formula (I), a salt of compound of formula (I) or an isomeric or polymorphic form thereof in combination with a further pharmaceutically active compound for the preparation of a pharmaceutical composition for the treatment, diagnosis, or prophylaxis of inflammatory diseases or conditions, is also subject of the present invention. The pharmaceutically active ingredients that can be used for the pharmaceutical compositions described herein are defined as the cosmetically active ingredients defined above. Dermatologically or pharmaceutically active ingredients can be identical to the cosmetically active ingredients as defined in this invention.

In particular, the present invention relates to the use of a compound of formula (I), a salt of compound of formula (I) or an isomeric or polymorphic form thereof for the preparation of a pharmaceutical composition for the treatment, diagnosis, or prophylaxis of inflammatory diseases or conditions, in particular chronic obstructive pulmonary disease (COPD), acute lung injury (ALI), cardiopulmonary bypass, acute respiratory distress syndrome (ARDS), septic shock, sepsis, psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma, and inflammatory bowel diseases (particularly Crohn's disease) and metastasis of cancers.

Other features of the invention will become apparent in the course of the following descriptions of exemplary embodiments, which are given for illustration of the invention and are not intended to be limiting thereof.

### Examples

### Example 1: Inhibition of molecular binding to selectins

### Sialyl Lewis^{X} Tyrosine Sulfate Assay (sLe^{X} TSA):

Compound of formula (I) is assayed on a molecular level for the ability to inhibit the binding of P- and L-selectin chimeric molecules to sLe^{X} and tyrosinesulfate residues linked to a polymeric matrix as a PSGL-1 substitute. IC₅₀-values are determined.

Microtiter plates are coated overnight in carbonate buffer pH9,6 with goat anti human Fc mAB (10 µg/ml). After washing in assay buffer (25 mM 4-(2-hydroxyethyl)-1-piperazineethanesulfonic acid (HEPES), 150 mM NaCl, 1 mM CaCl₂ pH7,4) and blocking (3% bovine serum albumin (BSA) in assay buffer) plates are incubated for 2 h at 37°C with human P-Selectin-IgG-chimera (0,61 nM respectively 150 ng/mL) or human L-Selectin-IgG-chimera (0,61 nM respectively 89 ng/mL). 5 µl of sLe^{X} -tyrosine sulfate polyacrylamide (1 mg/ml) carrying 15% sLe^{X}, 10% Tyrosine-sulfate and 5% biotin is complexed with 20 µl Streptavidin-Peroxidase solution (1 mg/ml) and 25 µl assay buffer without CaCl₂.

For use in the assay, the ligand complex is diluted 1:10000 in assay buffer and further diluted 1:1 with varying amounts of compound of formula (I) in assay buffer incl. 2% DMSO. This mixture is added to the wells precoated with L or P-selectin.

After incubation for 2 h at 37°C, wells are washed for six times with in assay buffer incl. 0,005% Polyoxyethylenesorbitan monolaurate (TWEEN 20), developed for 10-15 min with 20 µl 3,3',5,5'-tetramethylbenzidine (TMB)/H₂0₂ substrate solution and stopped with 20 µl 1 M H₂SO₄. Bound sLe^{X}-Tyrosine sulfate ligand complex is determined by measuring optical density at 450 nm vs. 620 nm in a Fusion alpha-FP reader (sold from Packard Bioscience, Dreieich, Germany). In addition to compound of formula (I) sLe^{X} is investigated in this assay employing the same procedure as applied for compound of formula (I).

| | IC₅₀ [µm] sLe^{X} TSA | |
|---|---|---|
| | P-Selectin | L-Selectin |
| sLe^{X} | >3600 | >3600 |
| compound of formula (I) | 189 | 1246 |

### Example 2: Inhibition of cell binding

### Flow Chamber Assay / Cell Adhesion and Rolling under Flow Conditions

To assess the capability of compound of formula (I) to inhibit cell binding under dynamic conditions resembling the flow in a blood vessel, flow chamber assays addressing/ testing binding of HL-60 cells / various cell lines to P-selectin, and E-selectin chimeric molecules are performed.

Cell attachment under flow conditions is determined using a parallel flow chamber system. A 35 mm polystyrene culture dish is coated for 1 h at room temperature with coating buffer (50 mM tris-(hydroxymethyl) aminomethane buffer (Tris), 150 mM NaCl, 2 mM CaCl₂; pH 7,4) containing human E- or P-selectin-IgG chimera at concentrations of 2,5 mg/ml or 10 mg/ml, respectively. After removal of the coating solution non specific binding sites are blocked for an additional hour with 1% BSA in coating buffer at room temperature.

After washing with assay buffer ("Roswell Park Memorial Institute 1640" (RPMI 1640) + 10 mM HEPES) the dish is fitted into a parallel plate laminar flow chamber (sold from Glycotech, Rockville, MD) and mounted on an inverted phase-contrast microscope (sold from Olympus, Hamburg, Germany) equipped with a CCD camera (JVC) that is connected to a PC. Employing a peristaltic pump (sold from Ismatec, Wertheim-Mondfeld, Germany) the recirculating system is equilibrated with assay buffer containing 125 mM compound of formula (I) or vehicle control (DMSO). Cells (4 million) are added to the chamber and allowed to distribute for 2 minutes at a high flow rate. The flow rate is then decreased resulting in a calculated flow shear of 1 dyne/cm² . Video sequences of 10 low power fields are digitally recorded after 5 minutes continuous flow. The percentage of inhibition is calculated from the mean number of cells per field that attached to the coated dish surface in the presence versus absence of compound of formula (I) of at independent experiments. In addition to compound of formula (I) sLe^{X} is investigated in this assay employing the same procedure as applied for compound of formula (I).

| | % inhibition of attachment of HL-60 cells | |
|---|---|---|
| | E-Selectin | P-Selectin |
| sLe^{X} | 32 | n.s. |
| compound of formula (I) | 30 | 36 |

| | | |
|---|---|---|
| *n.s. denotes " not significant"* | | |

### Example 3: Modulation of T-cells

For pre-incubation experiments Jurkat T-cells are incubated with compound of formula (I) or vehicle (DMSO) alone for 5 minutes at room temperature followed by two wash steps (assay buffer). Cell pellets are re-suspended in assay buffer and are used in the flow chamber assay as described in Example 2.

| | % inhibition of attachment of Jurkat cells (pre-treated) | |
|---|---|---|
| | E-Selectin | P-Selectin |
| compound of formula (I) | 30 | 39 |

### Example 4: Skin penetration

To predict passive human skin permeability of compound of formula (I) a parallel artificial membrane permeability assay is performed that mimics the main barrier properties of human stratum corneum (skin-PAMPA).

The filter of the donor compartment of a hydrophobic PVDF 96-well microtiter filter plate (Millipore, Schwalbach, Germany) is coated with a mixture of 70% silicone and 30% isopropyl myristate dissolved in hexane. After evaporation of solvent the donor compartment is filled with buffer (pH 4) containing compound of formula (I) and assembled to the acceptor compartment filled with buffer (pH 4). The assembled plate is incubated at room temperature for 16 hours. Absorbance of compound of formula (I) in the dissembled acceptor compartment is measured with a photometer (Spectramax, Molecular Devices, Sunnyvale, CA, USA). Permeation (% flux) of compound of formula (I) through the coated filter (artificial membrane) is calculated based on equilibrium absorbance.

| | Permeation [% flux] |
|---|---|
| Compound of formula (I) | > 60% |

## Claims

1. Use of a compound of formula (I), a salt of compound of formula (I) or an isomeric or polymorphic form thereof for the preparation of a cosmetic or dermatological composition.

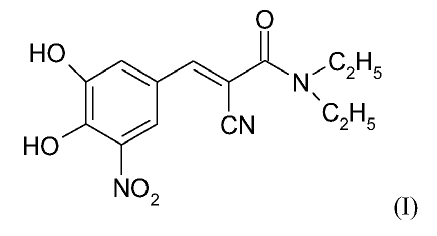

2. Cosmetic compositions comprising a compound of formula (I), a salt of compound of formula (I) or an isomeric or polymorphic form thereof and at least one cosmetically tolerable component.

3. Cosmetic compositions according to claim 2, comprising a compound of formula (I), a salt of compound of formula (I) or an isomeric or polymorphic form thereof and at least one cosmetically tolerable component for skin applications.

4. Cosmetic compositions according to one of the claims 2 or 3, comprising a compound of formula (I), a salt of compound of formula (I) or an isomeric or polymorphic form thereof and at least one further cosmetically active ingredient.

5. Dermatological compositions comprising a compound of formula (I), a salt of compound of formula (I) or an isomeric or polymorphic form thereof and at least one dermatologically tolerable component.

6. Dermatological compositions according to claim 5, comprising a compound of formula (I), a salt of compound of formula (I) or an isomeric or polymorphic form thereof and at least one dermatologically tolerable component for skin application.

7. Dermatological compositions according to one of the claims 5 or 6, comprising a compound of formula (I), a salt of compound of formula (I) or an isomeric or polymorphic form thereof and at least one further dermatologically or pharmaceutically active ingredient.

8. Process for the preparation of a cosmetic composition according to the claims 2, 3 or 4 by mixing a compound of formula (I), a salt of compound of formula (I) or an isomeric or polymorphic form thereof, at least one cosmetically tolerable component and eventually further cosmetically active ingredients.

9. Process for the preparation of a cosmetic composition according to claim 8 by mixing a compound of formula (I), a salt of compound of formula (I) or an isomeric or polymorphic form thereof, at least one cosmetically tolerable component and eventually further cosmetically active ingredients, wherein the composition includes from 0.01% to 20% by weight of compound of formula (I), a salt of compound of formula (I) or an isomeric or polymorphic form thereof, based on the total weight of the composition.

10. Process for the preparation of a dermatological composition according to claims 5, 6, or 7 by mixing a compound of formula (I), a salt of compound of formula (I) or an isomeric or polymorphic form thereof, at least one dermatologically tolerable component and eventually further pharmaceutically active ingredients.

11. Process for the preparation of a dermatological composition according to claim 10 by mixing a compound of formula (I), a salt of compound of formula (I) or an isomeric or polymorphic form thereof, at least one dermatologically tolerable component and eventually further pharmaceutically active ingredients, wherein the composition includes from 0.01% to 20% by weight of compound of formula (I), a salt of compound of formula (I) or an isomeric or polymorphic form thereof, based on the total weight of the composition.

12. Use of a compound of formula (I), a salt of compound of formula (I) or an isomeric or polymorphic form thereof for the preparation of a cosmetic composition for the treatment or prophylaxis of micro-inflammatory conditions.

13. Use of a compound of formula (I), a salt of compound of formula (I) or an isomeric or polymorphic form thereof according to claim 12 in combination with a further cosmetically active compound for the preparation of a cosmetic composition for the treatment or prophylaxis of micro-inflammatory conditions.

14. Use of a compound of formula (I), a salt of compound of formula (I) or an isomeric or polymorphic form thereof according to one of claims 12 or 13 for the preparation of a cosmetic composition for the treatment or prophylaxis of itching or skin-ageing caused by intrinsic factors.

15. Use of a compound of formula (I), a salt of compound of formula (I) or an isomeric or polymorphic form thereof for the preparation of a dermatological composition for the treatment or prophylaxis of micro-inflammatory diseases or conditions.

16. Use of a compound of formula (I), a salt of compound of formula (I) or an isomeric or polymorphic form thereof according to claim 15 in combination with a further dermatolo-gically or pharmaceutically active compound for the preparation of a dermatological composition for the treatment or prophylaxis of micro-inflammatory diseases or conditions.

17. Use of a compound of formula (I), a salt of compound of formula (I) or an isomeric or polymorphic form thereof according to one of claims 15 or 16 for the preparation of a dermatological composition for the treatment or prophylaxis of itching or skin-ageing caused by extrinsic factors.

18. Use of a compound of formula (I), a salt of compound of formula (I) or an isomeric or polymorphic form thereof according to one of claims 15 or 16 for the preparation of a dermatological composition for the treatment or prophylaxis of itching or skin ageing caused by a combination of intrinsic and extrinsic factors.

19. Use of a compound of formula (I), a salt of compound of formula (I) or an isomeric or polymorphic form thereof for the preparation of a dermatological composition for the treatment or prophylaxis of viral skin diseases and /or tumor diseases or conditions.

20. Use of a compound of formula (I), a salt of compound of formula (I) or an isomeric or polymorphic form thereof according to claim 19 in combination with a further dermatologically active compound for the preparation of a dermatological composition for the treatment or prophylaxis of viral skin diseases and /or tumor diseases or conditions.

21. Use of a compound of formula (I), a salt of compound of formula (I) or an isomeric or polymorphic form thereof according to one of claims 19 or 20 for the preparation of a dermatological composition for the treatment or prophylaxis of viral skin diseases caused by papilloma viruses, in particular human papilloma viruses (HPV), by herpes viruses, varicella zoster virus, or human herpes virus.

22. Use of a compound of formula (I), a salt of compound of formula (I) or an isomeric or polymorphic form thereof according to one of claims 19 or 20 for the preparation of a dermatological composition for the treatment or prophylaxis of viral skin diseases caused by papilloma viruses, in particular by HPV 1, 2, 3, 4, 5, 8, 9, 11, 12, 13, 14, 15, 16, 17, 18, 19-29, 31, 32, 34, 36-38, 46-50, 56, 58, by herpes viruses, in particular herpes simplex virus 1, herpes simplex virus 2, or human herpes virus 1, 2, 3, 4, 7, or 8.

23. Use of a compound of formula (I), a salt of compound of formula (I) or an isomeric or polymorphic form thereof according to one of claims 19, 20, 21, or 22 for the preparation of a dermatological composition for the treatment or prophylaxis of viral skin diseases, in particular genital warts, benign tumors of the skin and/or mucosa caused by papilloma viruses, in particular verrucae plantares, verrucae vulgares, verrucae planae juveniles, epidermodysplasia verruciformis, Condylomata acuminate, Condylomata plana, bowenoid papulosis, papilloma on the larynx and oral mucosa, focal epithelial hyperplasia, herpes labialis, Kaposi's sarcoma, varicella and shingles.

24. Use of a compound of formula (I), a salt of compound of formula (I) or an isomeric or polymorphic form thereof for the preparation of a pharmaceutical composition for the treatment, diagnosis, or prophylaxis of T-cell-mediated diseases or conditions.

25. Use of a compound of formula (I), a salt of compound of formula (I) or an isomeric or polymorphic form thereof according to claim 24 in combination with a further pharmaceutically active compound for the preparation of a pharmaceutical composition for the treatment, diagnosis, or prophylaxis of T-cell-mediated diseases or conditions.

26. Use of a compound of formula (I), a salt of compound of formula (I) or an isomeric or polymorphic form thereof according to one of claims 24 or 25 for the preparation of a pharmaceutical composition for the treatment, diagnosis, or prophylaxis of T-cell mediated disorders, in particular asthma bronchiale, psoriasis, atopische dermatitis, systemic lupus erythematosus (SLE), Sjörgen's syndrome, rheumatoid arthritis, encephalitis and in particular acute disseminated encephalomyelitis (ADEM), Addison's disease, antiphospholipid antibody syndrome (APS), aplastic anemia, autoimmune hepatitis, coeliac disease, inflammatory bowel disease and in particular Crohn's disease, diabetes mellitus (type 1), Goodpasture's syndrome, hyperthyroidism and in particular Graves' disease, Guillain-Barré syndrome (GBS; also called acute inflammatory demyelinating polyneuropathy, acute idiopathic polyradiculoneuritis, acute idiopathic polyneuritis and Landry's ascending paralysis), hypothyroidism and in particular Hashimoto's disease, idiopathic thrombocytopenic purpura, lupus erythematosus, multiple sclerosis, myasthenia gravis, opsoclonus myoclonus syndrome (OMS), optic neuritis, thyroiditis and in particular Ord's thyroiditis, pemphigus, polyarthritis, primary biliary cirrhosis, psoriasis, rheumatoid arthritis, Reiter's syndrome, Sjögren's syndrome, Takayasu's arteritis, temporal arteritis, warm autoimmune hemolytic anemia, vasulitis and in particular Wegener's granulomatosis, alopecia universalis, Behcet's disease, Chagas' disease, chronic fatigue syndrome, dysautonomia including postural orthostatic tachycardia syndrome (POTS), endometriosis, hidradenitis suppurativa, interstitial cystitis, neuromyotonia, sarcoidosis, scleroderma, ulcerative colitis, vitiligo, vulvodynia.

27. Use of a compound of formula (I), a salt of compound of formula (I) or an isomeric or polymorphic form thereof for the preparation of a pharmaceutical composition for the treatment, diagnosis, or prophylaxis of inflammatory diseases or conditions.

28. Use of a compound of formula (I), a salt of compound of formula (I) or an isomeric or polymorphic form thereof according to claim 27 in combination with a further pharmaceutically active compound for the preparation of a pharmaceutical composition for the treatment, diagnosis, or prophylaxis of inflammatory diseases or conditions.

29. Use of a compound of formula (I), a salt of compound of formula (I) or an isomeric or polymorphic form thereof according to one of claims 27 or 28 for the preparation of a pharmaceutical composition for the treatment, diagnosis, or prophylaxis of inflammatory diseases or conditions, in particular chronic obstructive pulmonary disease (COPD), acute lung injury (ALI), cardiopulmonary bypass, acute respiratory distress syndrome (ARDS), septic shock, sepsis, psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma, and inflammatory bowel diseases (particularly Crohn's disease) and metastasis of cancers.
